# EUROPÄISCHE PATENTSCHRIFT

(11) **EP 0 415 235 B1**
(45) Veröffentlichungstag und Bekanntmachung des Hinweises auf die Patenterteilung: **22.06.1994**
(21) Anmeldenummer: 90116000.2
(22) Anmeldetag: 21.08.1990
(51) Int. Cl.: C07C 67/08

(54) **Verfahren zur schonenden veresterung einer carbonsäure mit einer alkoholkomponente**
Process for carefully esterification of a carboxylic acid with an alcohol component
Procédé d'estérification ménagée d'un acide carboxylique avec un composant alcoolique

(30) Priorität: 29.08.1989 DE 3928564
(43) Veröffentlichungstag der Anmeldung: 06.03.1991
(73) Patentinhaber: Gesellschaft für Biotechnologische Forschung mbH (GBF), D-38124 Braunschweig (DE)
(72) Erfinder: Blankemeyer-Menge, Birgit, D-3300 Braunschweig (DE); Frank, Ronald, Dr., D-3300 Braunschweig (DE)
(74) Vertreter: Boeters, Hans Dietrich, Dr.

(56) Entgegenhaltungen:
- PATENT ABSTRACTS OF JAPAN vol. 7, no. 56 (C-155)(1201) 08 März 1983,& JP-A-57 206696 (EISAI K.K.) 18 Dezember 1982,
- TETRAHEDRON, [INCL. TETRAHEDRON REPORTS] vol. 44, no. 14, 1988, OXFORD GB, Seiten 4331-4338, TOSHIKI TANAKA et al.: "Solid phase synthesis of oligoribonucleotides using O-nitrobenzyl protection of 2'-hydroxyl via a phosphoriester approach"
- PATENT ABSTRACTS OF JAPAN vol. 6, no. 174 (C-123)(1052) 08 September 1982 & JP-A-57 088196
- TETRAHEDRON, [INCL. TETRAHEDRON REPORTS] vol. 36, 1980, OXFORD GB Seiten 3075 -3085; S.S. JONES ET AL.: "SYNTHESIS OF THE 3'-TERMINAL DECARIBONUCLEOSIDENONAPHOSPHATE OF YEAST ALANINE TRANSFER RIBONUCLEIC ACID"
- JOURNAL OF THE CHEMICAL SOCIETY, CHEMICAL COMMUNICATIONS. 1981, LETCHWORTH GBSeiten 336 - 340; ERIC ATHERTON ET AL.: "RACEMISATION OF ACTIVATED, URETHANE-PROTECTED AMINO-ACIDS BY P-DIMETHYLAMINOPYRIDINE. SIGNIFICANCE IN SOLID-PHASEPEPTIDE SYNTHESIS"
- JOURNAL OF THE AMERICAN CHEMICAL SOCIETY. vol. 85, no. 13, 05 Juli 1963,GASTON, PA US Seiten 2149 - 2154; R. B. MERRIFIELD: "SOLID PHASE PEPTIDESYNTHESIS. I. THE SYNTHESIS OF A TETRAPEPTIDE"
- TETRAHEDRON LETTERS. vol. 21, 1980, OXFORD GB Seiten 2265 - 2268; COLIN B.REESE ET AL.: "REACTION BETWEEN 1-ARENESULPHONYL-3-NITRO-1,2,4-TRIAZOLES ANDNUCLEOSIDE BASE RESIDUES. ELUCIDATION OF THE NATURE OF SIDE-REACTIONS DURINGOLIGONUCLEOTIDE SYNTHESIS. "

## Beschreibung

Die überwiegende Zahl von Naturstoffen, wie Antibiotika, Alkaloide, Proteine/Peptide, Coenzyme, Lipide, Saccharide/Oligosaccharide, Nucleinsäuren, Terpenoide, Steroide und Vitamine, sind komplexe, multifunktionelle organische Verbindungen, d. h. ihre Molekülstruktur weist nebeneinander mehrere Atomgruppen gleicher, ähnlicher und/oder unterschiedlicher Reaktionsfähigkeit auf. Die chemische Synthese oder Modifikation solcher Naturstoffe erfordert fast immer die regioselektive Umsetzung einer solchen Gruppe in Gegenwart der anderen Gruppen, ohne daß die Gesamt- oder Teilstruktur des Restmoleküls verändert oder zerstört werden darf.

Das im folgenden beschriebene erfindungsgemäße Verfahren wurde entwickelt, um unter sehr schonenden Bedingungen eine Carbonsäuregruppe (Carboxylgruppe = -COOH) mit einer Alkoholkomponente zu einem Ester umzusetzen, um empfindliche oder instabile Carbonsäuren mit gegebenenfalls empfindlichen oder instabilen Alkoholen befriedigend umsetzen zu können. Das Verfahren wird beispielhaft für die chemische Synthese von Peptiden aus geeignet geschützten Aminosäurederivaten erläutert.

Aminosäuren sind im allgemeinen chemische Verbindungen, die eine Aminogruppe (-NH₂), eine Carboxylgruppe (-COOH) und ggf. auch zusätzliche beliebige andere chemische Gruppen (Rₙ) enthalten. Eine spezielle Gruppe von Aminosäuren sind die natürlichen (sogenannten essentiellen) Aminosäuren, in denen die Amino- und Carboxylgruppe α-ständig sind und das chirale α-C-Atom ausschließlich in der L-Konfiguration vorliegt. Werden in Aminosäuren die Aminogruppe und ggf. die anderen Seitengruppen durch sogenannte Schutzgruppen in ihrer chemischen Reaktivität blockiert, so kann man ein solches Aminosäurederivat gezielt über die noch freie Carboxylgruppe mit einer Alkoholkomponente verestern. So ist z. B. für die chemische Synthese von Peptiden der Schutz der Carboxylfunktion der carboxyterminalen Aminosäure, also des ersten Peptidbausteins, durch Veresterung mit einem Alkoholderivat ein grundlegender Schritt. Bei der heute überwiegend durchgeführten modifizierten Festphasensynthese nach Merrifield (JACS, 85 (1963) 2149) verwendet man ein geeignetes Trägermaterial als C-terminalen Schutz. Ein solches Trägermaterial ist oft chemisch derart derivatisiert bzw. modifiziert, daß es an seiner Oberfläche Hydroxylgruppen trägt, mit denen sich geeignet geschützte Aminosäuren verknüpfen lassen. Für die beiden ersten Bausteine sei die Peptidsynthese durch das folgende Schema erläutert. 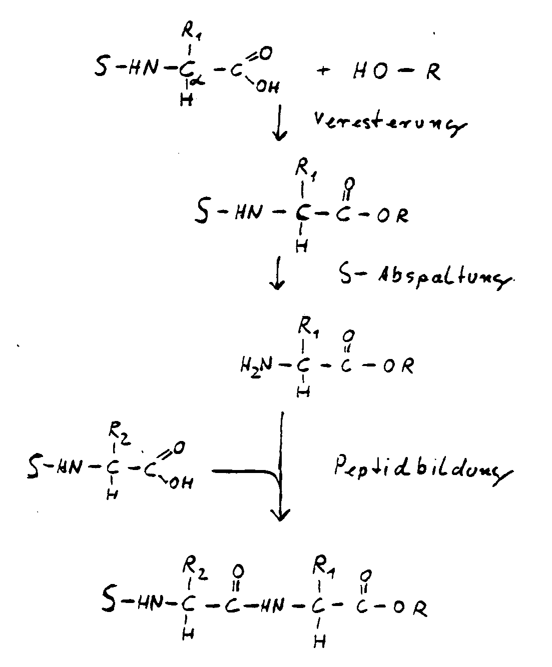
Die bekannte Veresterung erfordert schonende Reaktionsbedingungen, da viele geschützte Aminosäurederivate recht instabil sind. Der Literatur sind verschiedene Methoden zu entnehmen; vgl. J. Chem. Soc. Chem. Commun., (1981) 336; Tetrahedron Lett., 28 (1987) 6147; Liebigs Ann. Chem., (1987) 1031; und Int. J. Peptide Protein Res., 33 (1989) 386. Diese bekannten Methoden sind jedoch oft von Nebenreaktionen begleitet oder liefern oft unbefriedigende Ausbeuten. Die meisten Nebenreaktionen tretan auf, wenn starke Basen, beispielsweise Pyridin oder Dimethylaminopyridin (DMAP), als Katalysatoren eingesetzt werden. Beispielsweise ist es nicht möglich, das aus Tetrahedron, 36 (1980) 3075-3085 bekannte Verfahren, bei dem ein Phosphorsäurederivat in Gegenwart von 2,4,6-Mesitylen-1-sulfonyl-3-nitro-1,2,4-triazolid (MSNT) in Pyridin als Lösungsmittel verestert wird, zur Veresterung empfindlicher Carbonsäuren, wie Aminosäuren, heranzuziehen. So drückt sich die Empfindlichkeit von Aminosäuren aus in
- Dipeptidbildung durch Instabilität der Schutzgruppe S (beispielsweise Fluorenylmethoxycarbonyl (Fmoc)) und
- Razemisierung am chiralen C_{α}-Atom der Aminosäure.

Dementsprechend ist eine Verbesserung der bekannten Methoden erwünscht.

Dazu wird erfindungsgemäß ein Verfahren zur schonenden Veresterung einer Carbonsäure mit einer Alkoholkomponente in Gegenwart von 2,4,6-Mesitylen-1-sulfonyl-3-nitro-1,2,4-triazolid (MSNT) und einer Base vorgesehen, das dadurch gekennzeichnet ist, daß man in Gegenwart von N-Methylimidazol (MeIm) in einem aprotischen und nicht-basischen Lösungsmittel verestert.

Unter einer Alkoholkomponente sind niedermolekulare Komponenten, wie Schutzgruppen, hochmolekulare Komponenten, wie lösliche Träger oder Festphasenträger, zu verstehen. Hierzu und zum geeigneten Schutz von Aminosäuren vergleiche man Wünsch et al. in: Heuben-Weyl, Methoden der org. Chemie, 4. Auflage, Band 15, Thieme-Verlag, Stuttgart 1974. Für hydroxylgruppenhaltige Träger kann auf den Stand der Technik verwiesen werden.

Beispielsweise kann man eine geeignet geschützte Aminosäure, insbesondere eine Aminosäure mit einer α-ständigen geschützten Aminogruppe verestern.

Gemäß speziellen Ausführungsformen verwendet man die Aminosäure in Form eines ihrer Enantiomeren oder eine natürliche Aminosäuren. Das erfindungsgemäße Verfahren läßt sich für diese Ausführungsform durch folgendes Schema wiedergeben: 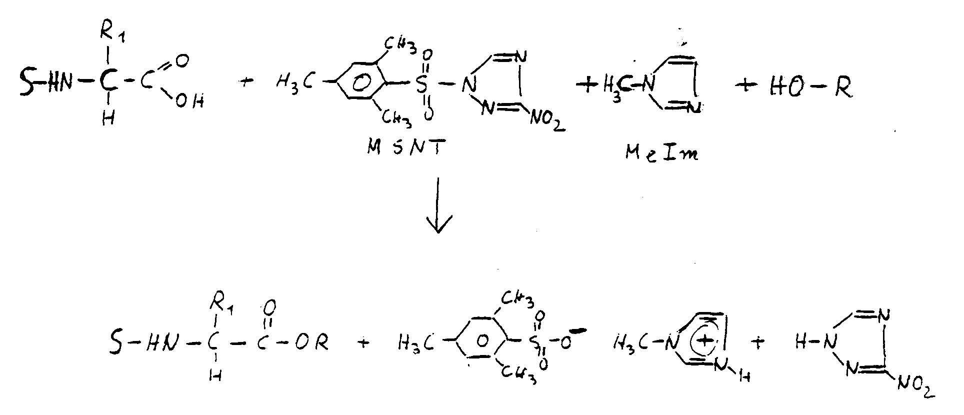
Das erfindungsgemäße Verfahren kann man in Dichlormethan, Chloroform oder THF als Lösungsmittel durchführen.

Beispielsweise kann man beim erfindungsgemäßen Verfahren bei einem molaren Verhältnis von Aminosäure:MSNT:MeIm wie 1: etwa 1: bis etwa 1 und vorzugsweise bis zu etwa 0.75 arbeiten.

Nachstehend wird die Erfindung durch Beispiele näher erläutert.

### Beispiel 1 und Vergleichsbeispiel 1

- DCC: = Dicyclohexylcarbodiimid
- DMAP: = Dimethylaminopyridin
- Fmoc: = Fluorenylmethoxycarbonyl
- MeIm: = N-Methylimidazol
- MSNT: = 2,4,6-Mesitylen-1-sulfonyl-3-nitro-1,2,4-triazolid
- NMM: = N-Methylmorpholin
- 1 eq: = mmol OH-Funktionen an eingesetzter Menge Trägermaterial

Es wurden verschiedene αN-Fmoc-geschützte Aminosäurederivate mit Cellulose (Trägermaterial) verestert, die mit p-Alkoxybenzylalkohol derivatisiert war; Frank & Döring, Tetrahedron, 44 (1988) 6031-6040. Die nach dem erfindungsgemäßen Verfahren erhaltenen Ergebnisse lassen sich der folgenden Tabelle Spalte A (Beispiel 1) entnehmen.

Die erfindungsgemäßen Versuche wurden ferner mit dem Stand der Technik verglichen; J. Chem. Soc. Chem. Commun., (1981) 336. Dazu wurden dieselben Aminosäuren mit Dicyclohexylcarbodiimid (DCC) als Kondensationsmittel und Dimethylaminopyridin (DMAP) sowie N-Methylmorpholin (NMM) als Basen mit der vorstehend genannten derivatisierten Cellulose verknüpft, da es sich bei dieser Standardreaktion um die gebräuchlichste Methode des Stands der Technik handelt. Die Ergebnisse lassen sich der folgenden Tabelle 1 Spalte B (Vergleichsbeispiel 1) entnehmen.

**Tabelle 1**

| Aminosäurederivat | A MSNT/MeIm | | B DCC/DMAP/NMM | |
|---|---|---|---|---|
| | Ausbeute [%] | Razemat [%] | Ausbeute [%] | Razemat [%] |
| Fmoc Asp(tBu) | 53 | 1.7 | 52 | 2.8 |
| Fmoc Cys(tBu) | 76 | 2.3 | 65 | 11.6 |
| Fmoc Phe | 68 | 0.7 | 69 | 2.0 |
| Fmoc Trp | 74 | < 0.2 | 63 | 0.8 |
| Fmoc Ile | 48 | < 0.2 | 26 | 3.0 |
| Fmoc Lys(Boc) | 72 | < 0.2 | 44 | 1.0 |
| A: 2 eq. Aminosäurederivat 2 eq. MSNT 1,5 eq. MeIm in Dichlormethan/Tetrahydrofuran 30 min Raumtemperatur B: 2 eq. Aminosäureanhydrid (*in situ* hergestellt aus 4 eq. Aminosäurederivat und 2 eq. DCC) 0,1 eq. DMAP 1 eq. NMM in Dimethylformamid 30 min Raumtemperatur | | | | |

Der Tabelle läßt sich entnehmen, daß sich erfindungsgemäß vergleichbar gute oder bessere Ausbeuten sowie eine deutliche geringere Razemisierungsrate erreichen lassen.

Wenn man beim erfindungsgemäßen Verfahren so wie bei der Methode des Stands der Technik 4 eq. Aminosäurederivat einsetzt, was einem gleichen Verbrauch an geschützter Aminosäure entspricht, lassen sich Ausbeuten von 80 bis 100 % erreichen.

### Beispiel 2 und Vergleichsbeispiel 2

Die erfindungsgemäßen Versuche des Beispiels 1 wurden mit der Ausnahme wiederholt, daß anstelle der derivatisierten Cellulose ebenso derivatisiertes Polystyrol verwendet wurde, wobei gleich gute Ergebnisse erhalten wurden. Zum Träger vergleiche Wang, JACS, 95 (1973) 1328-1333. Die Ergebnisse sind Tabelle 2 (A: Beispiel 2; B: Vergleichsbeispiel 2) zu entnehmen.

**Tabelle 2**

| Aminosäurederivat | A MSNT/MeIm | | B DCC/DMAP/NMM | |
|---|---|---|---|---|
| | Ausbeute [%] | Razemat [%] | Ausbeute [%] | Razemat [%] |
| Fmoc Asp(tBu) | 65 | nicht best. | 49 | nicht best. |
| Fmoc Phe | 59 | nicht best. | 55 | nicht best. |
| Fmoc Ile | 55 | nicht best. | 49 | nicht best. |
| A: 2 eq. Aminosäurederivat 2 eq. MSNT 1,5 eq. MeIm in Dichlormethan/Tetrahydrofuran 30 min Raumtemperatur B: 2 eq. Aminosäureanhydrid (*in situ* hergestellt aus 4 eq. Aminosäurederivat und 2 eq. DCC) 0,1 eq. DMAP 1 eq. NMM in Dimethylformamid 30 min Raumtemperatur | | | | |

### Vergleichsbeispiele 3 bis 4 und Beispiele 3 bis 4

Es wurde Fmoc-Leu-OH (AS) in Methylenchlorid in Gegenwart von 1-Mesitylensulfonyl-3-nitro-1,2,4-triazol (MSNT) mit Hilfe von Pyridin (Vergleichsbeispiele 3 und 4) und 1-Methylimidazol (MeIm) mit Cellulose verestert, die mit Alkoxybenzylalkohol beladen war. Einzelheiten sowie die Reaktionszeiten und Ausbeuten sind der folgenden Tabelle zu entnehmen:

| Beispiel | Vergleichsbeispiel | Fmoc-Leu-OH [Äqu.*] | MSNT [Äqu.*] | Base [Äqu.*] | RZ [min] | Ausbeute | |
|---|---|---|---|---|---|---|---|
| | | | | | | [» mol/F | [%*] |
| | 3 | 5 | 5 | 20 Pyridin | 30 | 1,8 | 23 |
| 3 | | 5 | 5 | 20 MeIm | 15 | 6,7 | 84 |
| | 4 | 5 | 10 | 20 Pyridin | 30 | 2,8 | 35 |
| 4 | | 5 | 10 | 20 MeIm | 15 | 7,2 | 90 |

| | | | | | | | |
|---|---|---|---|---|---|---|---|
| *Bezogen auf die Alkoxybenzylalkoholbeladung der eingesetzten Cellulose | | | | | | | |

Die Beispiele 3 und 4 belegen, daß MeIm eine sehr viel bessere Base als z. B. Pyridin ist.

## Patentansprüche

1. Verfahren zur schonenden Veresterung einer Carbonsäure mit einer Alkoholkomponente in Gegenwart von 2,4,6-Mesitylen-1-sulfonyl-3-nitro-1,2,4-triazolid (MSNT) und einer Base, dadurch *gekennzeichnet*, daß man in Gegenwart von N-Methylimidazol (MeIm) in einem aprotischen und nicht-basischen Lösungsmittel verestert.

2. Verfahren nach Anspruch 1, dadurch *gekennzeichnet*, daß man die Säure mit einem Hydroxylgruppen tragenden Festphasenträger verestert.

3. Verfahren nach einem der vorhergehenden Ansprüche, dadurch *gekennzeichnet*, daß man eine geeignet geschützte Aminosäure, insbesondere eine Aminosäure mit einer α-ständigen geschützten Aminogruppe verestert.

4. Verfahren nach einem der vorhergehenden Ansprüche, dadurch *gekennzeichnet*, daß man die Aminosäure in Form eines ihrer Enantiomeren oder daß man eine natürliche Aminosäure einsetzt.

5. Verfahren nach einem der vorhergehenden Ansprüche, dadurch *gekennzeichnet*, daß man in Dichlormethan, Chloroform oder THF als Lösungsmittel verestert.

6. Verfahren nach einem der vorhergehenden Ansprüche, dadurch *gekennzeichnet*, daß man bei einem molaren Verhältnis von Aminosäure:MSNT:MeIm wie 1: etwa 1: bis zu etwa 1 und vorzugsweise bis zu etwa 0.75 arbeitet.

## Claims

1. A process for the mild esterification of a carboxylic acid with an alcohol component in the presence of 2,4,6-mesitylene-1-sulphonyl-3-nitro-1,2,4-triazolide (MSNT) and a base, characterised in that the esterification is carried out in the presence of N-methylimidazole (MeIm) in an aprotic and non-basic solvent.

2. A process according to claim 1, characterised in that the acid is esterified using a solid-phase support carrying hydroxyl groups.

3. A process according to any one of the preceding claims, characterised in that a suitably protected amino acid, especially an amino acid having a protected amino group in the α-position, is esterified.

4. A process according to any one of the preceding claims, characterised in that the amino acid is employed in the form of one of its enantiomers, or a natural amino acid is employed.

5. A process according to any one of the preceding claims, characterised in that the esterification is carried out in dichloromethane, chloroform or THF as solvent.

6. A process according to any one of the preceding claims, characterised in that the reaction is carried out at a molar ratio of amino acid:MSNT:MeIm such as 1: about 1: up to about 1 and preferably up to about 0.75.

## Revendications

1. Procédé pour estérifier, dans des conditions ménagées, un acide carboxylique avec un composant alcool en présence de 2,4,6-mésitylène-1-sulfonyl-3-nitro-1,2,4-triazolide (MSNT) et d'une base, caractérisé en ce qu'on procède à l'estérification en présence de N-méthylimidazole (MeIm) dans un solvant aprotique et non basique.

2. Procédé selon la revendication 1, caractérisé en ce qu'on estérifie l'acide avec un support en phase solide portant des groupes hydroxyle.

3. Procédé selon l'une des revendications précédentes, caractérisé en ce qu'on estérifie un acide aminé convenablement protégé, en particulier un acide aminé comportant un groupe amino protégé en positions α.

4. Procédé selon l'une des revendications précédentes, caractérisé en ce qu'on utilise l'acide aminé sous forme de l'un de ses énantiomères, ou que l'on utilise un acide aminé naturel.

5. Procédé selon l'une des revendications précédentes, caractérisé en ce qu'on procède à l'estérification dans du dichlorométhane, du chloroforme ou du THF servant de solvant.

6. Procédé selon l'une des revendications précédentes, caractérisé en ce qu'on travaille selon un rapport molaire acides aminés:MSNT:MeIm de 1:environ 1:inférieur ou égal à environ 1, et de préférence inférieur ou égal à environ 0,75.
